Europäisches Patentamt

European Patent Office

Office européen des brevets

(19)

(11) Veröffentlichungsnummer: **0 071 995**
**B1**

(12) **EUROPÄISCHE PATENTSCHRIFT**

(45) Veröffentlichungstag der Patentschrift:
08.01.86

(51) Int. Cl.⁴: **A 61 K 9/10,** A 61 K 31/23

(21) Anmeldenummer: 82107128.9

(22) Anmeldetag: 06.08.82

(54) Fettemulsion für parenterale Ernährung.

(30) Priorität: 08.08.81 DE 3131460

(43) Veröffentlichungstag der Anmeldung:
16.02.83 Patentblatt 83/7

(45) Bekanntmachung des Hinweises auf die Patenterteilung:
08.01.86 Patentblatt 86/2

(84) Benannte Vertragsstaaten:
AT BE CH DE FR GB IT LI LU NL SE

(56) Entgegenhaltungen:
BE - A - 885 844
CA - A - 725 596
DE - A - 3 032 300

CHEMICAL ABSTRACTS, Band 74, Nr. 1, 4. Januar 1971,
Seite 132, Nr. 1665k, Columbus, Ohio, USA D. GUISARD
et al.: "Plasma clearance of synthetic emulsions of
triglycerides containing long-chain fatty acids and
medium-chain fatty acids"

(73) Patentinhaber: Intermedicat GmbH, Gerliswilstrasse 45,
CH-6020 Emmenbrücke (CH)

(72) Erfinder: Niemann, Wilhelm, Dr., Oberes Bachfeld 25,
D-3508 Melsungen (DE)
Erfinder: Nehne, Jörg, Dr., Weserring 7,
D-3501 Guxhagen (DE)

(74) Vertreter: von Kreisler, Alek, Dipl.-Chem. et al,
Deichmannhaus am Hauptbahnhof, D-5000 Köln 1 (DE)

## Beschreibung

Die Verwendung von Fettemulsionen in der Parenteralen Ernährung ist bei vielen Krankheitszuständen zu einem unverzichtbaren Bestandteil der medizinischen Therapie geworden.

Diese Fettemulsionen dienen dem Patienten als hochkalorische Energiequelle, wobei sie in der Regel in Kombination mit Aminosäuren- und/oder Kohlenhydrat-Infusionslösungen zur Anwendung kommen.

Bisher übliche und im Handel befindliche Fettemulsionen bestehen aus einer Fettkomponente, einem geeigneten Emulgator und einer oder mehrerer Komponenten wie z.B. Zuckeralkohole, die eine Isotonie der Lösung herbeiführen sollen.

Als Fettart werden ausschliesslich pflanzliche Öle eingesetzt, z.B. Baumwollsamenöl, Sojabohnenöl, Distelöl, deren Hauptbestandteil Triglyceride langkettiger gesättigter und ungesättigter Fettsäuren sind (LCT; $C_{16}$–$C_{18}$).

So wird in der kanadischen Patentschrift 725 596 ein Verfahren zur Herstellung von intravenös zu verabreichenden Fettemulsionen von pflanzlichen Ölen und Phosphatiden beschrieben, wonach eine wässrige Mischung aus Sojabohnenöl und Eiphosphatid emulgiert und anschliessend homogenisiert wird. In der belgischen Patentschrift 885 844 wird eine wässrige Fettemulsion zur intravenösen Injektion beschrieben, die aus Sojabohnenöl, Phospholipid des Eigelbs, Fettsäure mit 12 bis 20 C-Atomen oder einem pharmazeutisch unbedenklichen Salz davon und schliesslich aus Cholesterin besteht.

Daneben sind in den letzten Jahren auch Fettemulsionen experimentell zur Anwendung gekommen, die als Fettbestandteil mittelkettige Triglyceride (MCT; $C_8$–$C_{12}$) enthalten. Ausserdem sind experimentelle und klinische Studien mit verschiedenen Kombinationen MCT/LCT-haltiger Emulsionen durchgeführt worden.

Langkettige Triglyceride werden zwar gut vertragen, stehen aber nur zu einem geringen Teil als kurzfristige Energieträger zur Verfügung, da innerhalb 24 Stunden nur ca. 35% verwertet werden. Denn der Hauptanteil der i.v. zugeführten LCT wird bekanntlich zu Depotfett umgewandelt. Demgegenüber werden mittelkettige Triglyceride wesentlich rascher umgesetzt, da sie sehr schnell durch Betaoxydation verstoffwechselt und nicht als Depotfett gespeichert werden.

So sind aus Chemical Abstracts, Band 74, Seite 132 Nr. 1665k kinetische Abbauversuche mit zwei künstlichen Emulsionen für parenterale Ernährung aus Triglyceriden mit langkettigen ($C_{14}$–$C_{18}$) und mittelkettigen ($C_6$–$C_{12}$) Fettsäuren, die an Hunde verabreicht wurden, bekannt. Sie zeigen, dass die clearance von Fettsäuren mit mittlerer Kettenlänge viel schneller war (30 Minuten) als die von langkettigen Säuren (90 Minuten), was eine viel schnellere Umwandlung wiedergibt.

Die Nachteile bei i.v.-Gabe von mittelkettigen Triglyceriden bestehen jedoch in einer schlechteren Verträglichkeit, die sich in Hämolyse von roten Blutkörperchen, in einem narkotisierenden Effekt, einem Anstieg der freien Fettsäuren und der Ketonkörper äussert. Diese Symptome sind wahrscheinlich durch eine rasche Freisetzung der Fettsäuren aus dem MCT hervorgerufen.

Von SAILER wurden jedoch interessanterweise Kombinationen von LCT und MCT klinisch mit gutem Ergebnis getestet, wobei verschiedene Mengenverhältnisse der beiden Triglyceride zum Einsatz kommen.

Alle MCT-haltigen Emulsionen, die bisher in der Literatur beschrieben sind, enthalten als Emulgator Sojaphosphatide oder Gelatine.

Obwohl diese Fettemulsionen sich im allgemeinen bei klinisch üblicher Dosierung als sehr gut verträglich erwiesen haben, stellten sich bei toxikologischen Untersuchungen an Versuchstieren bei sehr hohen Dosierungen entscheidende Unterschiede heraus im Vergleich zu Fettemulsionen, die nur LCT als Fettkomponente enthielten. Dies wird aus Folgendem ersichtlich.

An 5 aufeinanderfolgenden Tagen wurden je 6 Bastardkaninchen verschiedene Emulsionen mit einer Dosierung von 6 g Fett/kg KG und einer Infusionsgeschwindigkeit von 30 ml/h verabreicht.

Tabelle 1

| Fettemulsion | Mortalität [%] |
| --- | --- |
| Fettemulsion I 20% (LCT mit Sojaphosphatid) | 0 |
| Fettemulsion II 20% (LCT mit Eiphosphatid) | 0 |
| Emulsionen mit 20% Fett, je 10% LCT und 10% MCT (mit Sojaphosphatid) | 66,7 |

Wie aus der Tabelle ersichtlich, bestehen hinsichtlich der Verträglichkeit zwischen den Emulsionen signifikante Unterschiede zu ungunsten der MCT-haltigen Infusion: während die Fettemulsionen mit nur LCT keine Mortalität verursachen, steigt die Mortalität bei Verwendung eines Gemisches von LCT und MCT sehr stark auf 66,7% an.

Diese signifikanten Unterschiede werden weiter bestätigt durch Versuche zur akuten Toxizität, die in der folgenden Tabelle aufgeführt sind, wobei alle $LD_{50}$-Werte in ml/kg Körpergewicht angegeben sind.

Tabelle 2

| Fettemulsion | Maus | LD$_{50}$-Werte Ratte | Kaninchen |
|---|---|---|---|
| Fettemulsion I 20% (LCT mit Sojaphosphatid) | 102 ± 8,7 | 100 ± 11,1 | 61,5 ± 8,3 |
| Fettemulsion II 20% (LCT mit Eiphosphatid) | 110 ± 7,4 | 92 ± 12,4 | 64,2 ± 7,9 |
| Emulsion mit 20% Fett, je 10% LCT und 10% MCT (mit Sojaphosphatid) | 76,4 ± 6,8 | 70,7 ± 9,5 | 48 ± 5,4 |

Auch hier sind die LD$_{50}$-Werte für das LCT plus MCT-Gemisch sehr viel schlechter als für LCT allein.

Überraschenderweise wurde nun gefunden, dass bei einer Kombination von Eiphosphatid mit einer LCT/MCT-Mischung von (z.B. je 10 Gew.-%) die Verträglichkeit nach mehrmaliger Gabe am Kaninchen sowie nach einmaliger Gabe an Mäusen, Ratten und Kaninchen erheblich verbessert ist, so dass keine Unterschiede mehr zu den nur LCT-haltigen Emulsionen festgestellt werden können. Darauf basiert die vorliegende Erfindung.

Demgemäss betrifft die Erfindung eine isotone LCT/MCT-Emulsion (langkettige Triglyceride mit 16–18 C-Atomen/mittelkettige Triglyceride mit 8–12 C-Atomen) zur parenteralen Anwendung mit einem Gesamtfettgehalt von 3 bis 30% und einem LCT/MCT-Verhältnis (langkettige Triglyceride mit 16–18 C-Atomen/mittelkettige Triglyceride mit 8–12 C-Atomen) zwischen 4:1 und 1:4 sowie einem Gehalt an physiologisch unbedenklichen mehrwertigen Alkoholen, die dadurch gekennzeichnet ist, dass sie als Emulgator Eiphosphatid enthält.

Die Menge des zur Emulsion verwendeten Eiphosphatid-Emulgators liegt – bezogen auf den Fettgehalt der Emulsion – zwischen 5 und 15%. Der Gesamtfettgehalt der isotonen LCT/MCT Öl-Wasser-Emulsion liegt zwischen 5 und 30%. Die Prozentangaben beziehen sich auf das Gewicht.

Mit den erfindungsgemässen Emulsionen wurden die vorstehend genannten Versuche bezüglich der Mortalität und der LD$_{50}$-Werte durchgeführt und die Ergebnisse in gleicher Weise wie in den Tabellen 1 und 2 dargestellt.

So wurde einerseits nach 5maliger Gabe von 6 g Fett/kg Körpergewicht und einer Infusionsgeschwindigkeit von 30 ml/Stunde bei 6 Bastardkaninchen keine Mortalität festgestellt (vgl. hierzu Tabelle 1), und andererseits wurden als LD$_{50}$-Werte gefunden: Maus 115±12,9 ml/kg KG; Ratte 104±13,1 ml/kg KG; Kaninchen 70±6,7 ml/kg KG (vgl. hierzu Tabelle 2). Aus diesen Werten geht die überraschende Überlegenheit der erfindungsgemässen LCT/MCT-Emulsion hervor.

Vorteilhaft für die Verträglichkeit und Stabilität solcher Emulsionen sind ausser Eiphosphatid weitere Zusätze von Salzen, besonders von Alkalisalzen langkettiger Fettsäuren, Cholesterin und Cholesterinderivate, die einzeln oder in Kombination miteinander in Mengen von 0 bis 5 Gew.-% enthalten sind. Als Beispiele langkettiger Fettsäuren können Palmitinsäure, Stearinsäure, Ölsäure, Linolsäure und Linolensäure genannt werden. Bevorzugt werden die Natriumsalze langkettiger Fettsäuren und auch Cholesterin oder Cholesterinester langkettiger Fettsäuren in einer Menge von 0,005 bis 0,1 Gew.-%, bezogen auf die Gesamtemulsion verwendet.

Die bei tierexperimentellem und auch bei klinischem Einsatz beobachtete gute Verträglichkeit sowie auch die nur geringfügigen Veränderungen biochemischer und hämotologischer Parameter, wie z.B. Triglyceride, Cholesterin, Bilirubin, β-Hydroxybutyrat, Lactat, Acetacetat bzw. Erythrozyten, Leukozyten, Thrombozyten, Hämoglobin, Hämatokrit, zeigen, dass die Kombination von MCT/LCT mit Eiphosphatid alle Vorteile der einzelnen Triglyceride vereinigt, ohne deren Nachteile erkennen zu lassen.

Folgende Herstellungsbeispiele erläutern die Erfindung:

Beispiel 1

Zu 1 kg einer Mischung, bestehend aus gleichen Anteilen Oleum Sojae fract. und Triglycerida mediocatenalia, werden 120 g gereinigte Eigelb-Phospholipide unter Verwendung eines schnelllaufenden Rührers (z.B. Ultra-Turrax) zugesetzt. Der Ansatz kann auf Temperaturen bis zu 75 °C erwärmt werden. Nach Vorliegen einer Lösung bzw. einer gleichmässigen Dispersion werden 250 g Glycerolum und 4 l Aqua ad iniectabilia unter Rühren zugefügt und grob voremulgiert. Anschliessend wird die entstandene Emulsion in einem Hochdruckhomogenisator bei einem Druck von 400 kg/cm$^2$ homogenisiert. Die entstandene feine Emulsion wird mit Aqua ad iniectabilia auf ein Volumen von 10 l aufgefüllt, in Glasflaschen geeigneter Qualität abgefüllt und nach allgemein bekanntem Verfahren hitzesterilisiert. Die Teilchen der sterilen und pyrogenfreien Emulsion sind kleiner 1 μm.

Beispiel 2

Zu 2 kg einer Mischung, bestehend aus gleichem Anteilen Oleum Sojae fract. und Triglycerida mediocatenalia, werden 120 g gereinigte Eigelb-Phospholipide und 5 g Natriumstearat unter Verwendung eines schnellaufenden Rührers (z.B. Ultra-Turrax) zugesetzt. Der Ansatz kann auf Temperaturen bis zu 75 °C erwärmt werden. Nach Vorliegen einer Lösung bzw. einer gleichmässigen Dispersion werden 250 g Glycerolum und 4 l Aqua ad iniectabilia unter Rühren zugefügt und grob voremulgiert. Anschliessend wird die entstandene Emulsion in einem Hochdruckhomogenisator bei einem Druck von 400 kg/cm² homogenisiert. Die entstandene feine Emulsion wird mit Aqua ad iniectabilia auf ein Volumen von 10 l aufgefüllt, in Glasflaschen geeigneter Qualität abgefüllt und nach allgemein bekanntem Verfahren hitzesterilisiert. Die Teilchen der sterilen und pyrogenfreien Emulsion sind kleiner 1 µm.

Beispiel 3

Zu 1 kg einer Mischung, bestehend aus 250 g Triglycerida mediocatenalia und 750 g Oleum Sojae fract., werden 120 g Eigelb-Phospholipide und 4 g Cholesterin unter Verwendung eines schnellaufenden Rührers (z.B. Ultra-Turrax) zugesetzt. Der Ansatz kann auf Temperaturen bis zu 75 °C erwärmt werden. Nach Vorliegen einer Lösung bzw. einer gleichmässigen Dispersion werden 250 g Glycerolum und 4 l Aqua ad iniectabilia unter Rühren zugefügt und grob voremulgiert. Anschliessend wird die entstandene Emulsion in einem Hochdruckhomogenisator bei einem Druck von 400 kg/cm² homogenisiert. Die entstandene feine Emulsion wird mit Aqua ad iniectabilia auf ein Volumen von 10 l aufgefüllt, in Glasflaschen geeigneter Qualität abgefüllt und nach allgemein bekanntem Verfahren hitzesterilisiert. Die Teilchen der sterilen und pyrogenfreien Emulsion sind kleiner 1 µm.

Beispiel 4

Zu 1 kg einer Mischung, bestehend aus 250 g Triglycerida mediocatenalia und 750 g Oleum Sojae fract., werden 120 g Eigelb-Phospholipide, 5 g Natriumpalmitat und 4 g Cholesterin unter Verwendung eines schnellaufenden Rührers (z.B. Ultra-Turrax) zugesetzt. Der Ansatz kann auf Temperaturen bis zu 75 °C erwärmt werden. Nach Vorliegen einer Lösung bzw. einer gleichmässigen Dispersion werden 250 g Glycerolum und 4 l Aqua ad iniectabilia unter Rühren zugefügt und grob voremulgiert. Anschliessend wird die entstandene Emulsion in einem Hochdruckhomogenisator bei einem Druck von 400 kg/cm² homogenisiert. Die entstandene feine Emulsion wird mit Aqua ad iniectabilia auf ein Volumen von 10 l aufgefüllt, in Glasflaschen geeigneter Qualität abgefüllt und nach allgemein bekanntem Verfahren hitzesterilisiert. Die Teilchen der sterilen und pyrogenfreien Emulsion sind kleiner 1 µm.

## Patentansprüche

1. Isotone LCT/MCT-Emulsion (langkettige Triglyceride mit 16–18 C-Atomen/mittelkettige Triglyceride mit 8–12 C-Atomen) zur parenteralen Anwendung mit einem Gesamtfettgehalt von 3–30% und einem LCT/MCT-Verhältnis (langkettige Triglyceride mit 16–18 C-Atomen/mittelkettige Triglyceride mit 8–12 C-Atomen) zwischen 4:1 und 1:4 sowie einem Gehalt an physiologisch unbedenklichen mehrwertigen Alkoholen, dadurch gekennzeichnet, dass die Emulsion als Emulgator Eiphosphatid enthält.

2. LCT/MCT-Emulsion gemäss Anspruch 1, dadurch gekennzeichnet, dass die Menge des zur Emulsion verwendeten Eiphosphatid-Emulgators – bezogen auf den Fettgehalt der Emulsion – zwischen 5 und 15% variiert.

3. LCT/MCT-Emulsion gemäss Ansprüchen 1 und 2, dadurch gekennzeichnet, dass der Gesamtfettgehalt der Öl/Wasser-Emulsion zwischen 5 und 30% variiert.

4. LCT/MCT-Emulsion gemäss Ansprüchen 1–3, dadurch gekennzeichnet, dass als zusätzliches Emulgierhilfsmittel die Na-Salze langkettiger Fettsäuren in einer Menge von 0,005 bis 0,1 Gew.-% – bezogen auf die Gesamtemulsion – verwendet werden.

5. LCT/MCT-Emulsion gemäss Ansprüchen 1–4, dadurch gekennzeichnet, dass als zusätzliches Emulgierhilfsmittel Cholesterin oder Cholesterinester langkettiger Fettsäuren in einer Menge von 0,005 bis 0,1 Gew.-% – bezogen auf die Gesamtemulsion – verwendet werden.

## Revendications

1. Emulsion LCT/MCT (triglycérides à longue chaîne ayant 16–18 atomes de carbone/triglycérides à chaîne moyenne ayant 8–12 atomes de carbone) isotonique, pour utilisation par voie parentérale, ayant une teneur totale en graisses de 3–30% et un rapport LCT/MCT (triglycérides à longue chaîne ayant 16–18 atomes de carbone/triglycérides à chaîne moyenne ayant 8–12 atomes de carbone) compris entre 4:1 et 1:4, et contenant des polyalcools physiologiquement non-toxiques, caractérisée en ce que l'émulsion contient comme émulsionnant un phosphatide d'œuf.

2. Emulsion LCT/MCT selon la revendication 1, caractérisée en ce que la concentration de l'émulsionnant phosphatide d'œuf utilisée pour l'émulsion est, sur la base de la teneur de l'émulsion en graisses, comprise entre 5 et 15%.

3. Emulsion LCT/MCT selon les revendications 1 et 2, caractérisée en ce que la teneur en graisses totale de l'émulsion du type aqueux est comprise entre 5 et 30%.

4. Emulsion LCT/MCT selon les revendications 1 à 3, caractérisée en ce qu'on utilise comme auxiliaire d'émulsification supplémentaire les sels de Na d'acides gras à longue chaîne, à une concentration de 0,005 à 0,1% en poids sur la base de l'émulsion totale.

5. Emulsion LCT/MCT selon les revendications 1 à 4, caractérisée en ce qu'on utilise comme auxiliaire d'émulsification supplémentaire du cholestérol ou un ester de cholestérol d'acides gras à longue chaîne, à une concentration de 0,005 à 0,1% en poids sur la base de l'émulsion totale.

## Claims

1. An isotonic emulsion composition adapted for parenteral use comprising triglycerides of long-chain fatty acids having from 16 to 18 carbon atoms (LCT) and triglycerides of medium-chain fatty acids having from 8 to 12 carbon atoms (MCT), wherein the total fat content is from 3 to 30% and the ratio LCT/MCT (triglycerides of long-chain fatty acids having from 16 to 18 carbon atoms/triglycerides of medium-chain fatty acids having from 8 to 12 carbon atoms) is from 4:1 to 1:4, and a content of physiologically accetable polyvalent alcohols, characterized in that the emulsion contains an egg phosphatide as the emulsifier.

2. The LCT/MCT emulsion composition according to claim 1, characterized in that the amount of the egg phosphatide emulsifier used for emulsification varies between 5 and 15%, based on the fat content of the emulsion.

3. The LCT/MCT emulsion composition according to claims 1 and 2, characaterized in that the total fat content of the oil/water emulsion varies between 5 and 30%.

4. The LCT/MCT emulsion composition according to claims 1 to 3, characterized in that sodium salts of long-chain fatty acids in an amount of from 0.005 to 0.1% by weight, based on the total emulsion, are used as additional emulsifying aids.

5. The LCT/MCT emulsion composition according to claims 1 to 3, characterized in that cholesterol or cholesterol esters of longchain fatty acids in an amount of from 0.005 to 0.1% by weight, based on the total emulsion, are used as additional emulsifying aids.